Europäisches Patentamt

European Patent Office

Office européen des brevets

(11) Publication number: **0 267 015 B1**

(12) **EUROPEAN PATENT SPECIFICATION**

(45) Date of publication of patent specification: **02.09.92**

(51) Int. Cl.5: **A61K 47/00**, A61K 37/36, A61K 37/02, A61K 9/06, A23C 11/00, A61L 17/00

(21) Application number: **87309748.9**

(22) Date of filing: **04.11.87**

(54) Stabilized compositions containing epidermal growth factor.

(30) Priority: **05.11.86 US 927627**
**18.09.87 US 96455**

(43) Date of publication of application:
**11.05.88 Bulletin 88/19**

(45) Publication of the grant of the patent:
**02.09.92 Bulletin 92/36**

(84) Designated Contracting States:
**AT BE CH DE ES FR GB IT LI LU NL SE**

(56) References cited:
**EP-A- 0 140 998**
**EP-A- 0 193 917**
**GB-A- 2 160 528**
**US-A- 4 179 497**

(73) Proprietor: **ETHICON INC.**
**U.S. Route 22**
**Somerville New Jersey 08876(US)**

(72) Inventor: **Finkenaur, Amy L.**
**10 Steven Avenue**
**Somerville, NJ 08876(US)**

(74) Representative: **Jones, Alan John et al**
**CARPMAELS & RANSFORD 43 Bloomsbury Square**
**London, WC1A 2RA(GB)**

Rank Xerox (UK) Business Services

EP 0 267 015 B1

## Description

The invention relates to a means for stabilizing compositions containing polypeptides, and in particular polypeptide growth factors such as epidermal growth factor.

Human epidermal growth factor (EGF) is a 53 amino acid polypeptide growth factor that has mitogenic activity for a number of kinds of cells, including epithelial and mesenchymal cells. Variants of the human EGF polypeptide have been reported, such as the 52 amino acid gamma-urogastrone. Epidermal growth factor exhibits epidermal growth promoting activity and gastric acid secretion inhibiting activity, and is therefore useful as a medicament. It has been found that epidermal growth factor loses biological activity in the presence of moisture. This is disadvantageous because such loss of activity makes it impractical to store aqueous preparations of epidermal growth factor for extended periods of time. This invention provides a means for reducing the loss of activity of polypeptide growth factors, including epidermal growth factor, in the presence of moisture.

The present invention provides a method for stabilizing medicinal compositions containing growth factors against loss of biological activity in the presence of moisture. The method comprises incorporating in said compositions a water soluble cellulose polymer.

Use of pharmaceutical and chemical compositions containing cellulose derivatives has been described. For example, Errede et al., in U.S. Patent No. 4,373,519, disclose a wound dressing including an absorbent such as cellulose material (which may be carboxymethyl-cellulose), wherein the wound dressing may contain various medicaments such as epidermal growth factor.

Hess et al. in U.S.Patent No. 3,923,599, disclose plant enzyme formulations which may include a cellulose derivative. Straub, in U.S. Patent No. 3,927,209, discloses a para-influenza-3-virus composition that may include methylcellulose as a dispersing agent. Dworschack et al., in U.S. Patent No. 3,933,588, disclose enzymes immobilized on a cellulose derivative (DEAE cellulose) containing a quaternary ammonium compound as a stabilizer. Diehl et al., in U.S. Patent No. 4,011,169 disclose an enzyme-containing detergent composition containing an aminated starch or cellulose as a stabilizer.

EP-A-0 193 917 discloses compositions of water soluble/dispersible carbohydrate polymers and biologically active macromolecules of growth hormones. Such compositions are parentally administered to animals.

Straub, in U.S. Patent No. 4,188,375, discloses aqueous vaccine preparations that may contain methylcellulose as a dispersing agent. "Polysaccharides" are disclosed as "stabilizers", but it is not disclosed for what instability problem the stabilizers are added.

GB-A-2 160 528 discloses the stabilization of proteinaceous bioactive substances (eg lymphokine and peptide hormone) in dry solid by the presence of a polysaccharide composed of repeating maltotriose units.

Akagi et al., in European Patent Application Publication No. 0 150 067, disclose gamma-interferon stabilized with dextran or hydroxyethyl starch.

Jacobsen et al., in U.S. Patent No. 4,540,506, disclose drain-cleaning formulations containing enzymes, which may contain hydroxyethyl cellulose as a thickener.

Arakawa and Timasheff, in Biochemistry 1982, Vol. 21, pages 6536-6544, disclose the use of sugars to stabilize proteins in aqueous media. Gekko, K. et al., J. Biochem (1981) 90:30-60, disclose that sugar alcohols, such as glycerol, sorbitol and mannitol, are stabilizing agents for proteins.

European Patent Application Publication No. 140,998 describes an ophthalmic preparation containing human epidermal growth factor and a diluent such as a buffer solution or an ointment base. The preparation is described as being effective to cure keratitis, corneal erosion, corneal infiltration or corneal ulcers.

## Summary of the Invention

The present invention provides an aqueous medicinal composition containing a specified polypeptide growth factor and a water soluble cellulose polymer sufficient to stabilize the polypeptide growth factor against loss of biological activity. In a preferred embodiment, the growth factor is epidermal growth factor. A method for stabilizing an aqueous medicinal composition containing a specified polypeptide growth factor as an active ingredient comprises incorporating into the composition an amount of a water soluble cellulose polymer sufficient to stabilize the growth factor against loss of biological activity in the presence of water.

## Detailed Description of the Invention

The invention resides in the provision of stabilized compositions containing polypeptide growth factors, preferably, human epidermal growth factor (hEGF). Epidermal growth factor (EGF) and hEGF are known

2

compositions that are either isolated from natural sources or are produced using recombinant DNA techniques. The following references describe epidermal growth factor, hEGF, and/or processes for isolating them from natural sources or producing them from rDNA techniques:

Camble et al., U.S. Patent No. 3,917,824

Cohen et al., U.S. Patent No. 3,948,875

Nishimura et al., U.S. Patent No. 4,528,186

Bell, Published PCT patent application WO 85/00369

Urdea et al., Proc. Natl. Acad. Sci. USA 80, 7461-7465 (1983)

Hollenberg, "Epidermal Growth Factor-Urogastrone, A Polypeptide Acquiring Hormonal Status", Acad. Press, Inc., N.Y. (1979) pp. 90-132

Carpenter, "Epidermal Growth Factor" in: Handbook of Experimental Pharmacology, Vol. 57, Baserga, ed.

Lawn et al., "Cell" (1978) 15:1157-1174

Savage et al., "J. Biol. Chem." (1972) 247:7612-7621

As used in this application, "epidermal growth factor" is intended to include the class of polypeptides that have biological activity similar to that exhibited by the natural human epidermal growth factor polypeptide as measured in recognized bioassays, such as the EGF receptor binding assay described hereinbelow, and which have certain conserved amino acid residues and common positioning of disulfide bonds, as discussed by Carpenter et al. in "Epidermal Growth Factor, Its Receptor, and Related Proteins", Experimental Cell Research 164, (1986) 1-10. Thus, "epidermal growth factor" includes the hEGF produced by recombinant DNA techniques described by Bell, op. cit. supra, mouse EGF ("mEGF") isolated from the submaxillary glands of mice (see, for example, Cohen et al., op. cit. supra), rat EGF, and natural human epidermal growth factor, which may be isolated from human urine as described by Nishimura et al., op. cit. supra, and bioactive derivatives and related polypeptides of any of the foregoing, including precursors that are transformed into active epidermal growth factor in situ by proteolytic processing. Human epidermal growth factor, including hEGF produced by recombinant DNA techniques, is preferred for use in the invention.

Transforming growth factor-alpha (TGF-α), a 50 amino acid polypeptide, and vaccinia virus growth factor (VGF), a 140 amino acid polypeptide, are both mitogenic and have substantial sequence homology to epidermal growth factor and all three appear to bind to and activate a common tyrosine kinase receptor. Because of their polypeptide nature, mitogenic activity and the substantial sequence homology between these molecules, it is contemplated that TGF-α and VGF may also be stabilized in the composition of the present invention. Additionally, the following polypeptide growth factors having mitogenic activity will also be stabilized in the composition of the present invention. These are basic fibroblast growth factor (b-FGF), acidic fibroblast growth factor (a-FGF), transforming growth factor-beta (TGF-B), angiogenin, nerve growth factor (NGF), insulin-like growth factor-I (IGF-I), insulin-like growth factor II (IGF-II) and platelet derived growth factor (PDGF). The fibroblast growth factors and angiogenin, as well as the transforming growth factors and epidermal growth factor, also have angiogenic activity. It is preferred that the growth factor be prepared by recombinant DNA techniques. As used herein, the phrase "growth factor" does not include the so called hematopoietic growth factors such as erythropoietin and the colony stimulating factors.

The cellulose stabilizers that are used in the invention are water-soluble etherified cellulose derivatives such as alkyl celluloses, hydroxyalkyl celluloses and alkylhydroxyalkyl celluloses, for example methylcellulose, hydroxyethyl cellulose, carboxymethyl cellulose, hydroxypropyl methylcellulose, and hydroxypropyl cellulose. Methylcellulose and the hydroxyalkyl cellulose derivatives such as hydroxypropyl cellulose, hydroxyethyl cellulose, and hydroxypropyl methylcellulose are preferred.

The solubility of the cellulose derivatives is determined by the degree of substitution (D.S.) of ether groups and the stabilizing derivatives useful in the present invention should have a sufficient quantity of such ether groups per anhydroglucose unit in the cellulose chain to render the derivatives water soluble. A degree of ether substitution (D.S.) of at least 0.35 ether groups per anhydroglucose unit is generally sufficient. Additionally, the cellulose derivatives may be in the form of alkali metal salts, for example, the Li, Na, K or Cs salts.

The stabilized compositions and ophthalmic formulations of the present invention may contain other polypeptide growth factors in admixture with the EGF. In particular, EGF may be combined with one or more of the following: basic fibroblast growth factor, acidic fibroblast growth factor, transforming growth factor-beta, transforming growth factor-alpha, vaccinia growth factor, angiogenin, nerve growth factor, insulin-like growth factor and platelet derived growth factor.

The compositions that are stabilized in accordance with the invention are compositions containing a polypeptide growth factor, such as EGF, either in an aqueous medicinal composition such as a gel, solution, suspension, or dispersion, in which is dissolved an effective amount of the water-soluble cellulose polymer.

The exact amount of the cellulose polymer to be used in specific cases will vary, depending on factors such as presence or absence of other materials in the formulation, specific nature of the particular growth factor used, concentration of the growth factor and type of formulation.

Expressed in terms of an aqueous formulation containing EGF (either an initial formulation or a formulation that has been reconstituted following dehydration) and a cellulose derivative, the effective amount of the cellulose derivative will usually be at least 0.05 weight per cent, based on weight of the whole composition. The maximum amount used is not at all critical, and will be in part determined by the type of formulation. For instance, an aqueous eye drop formulation will usually use an amount of the cellulose derivative within the range of from about 0.05 to about 3 weight per cent. In a gel, in which a relatively small amount of water might be used, the cellulose derivative can be the major constituent of the formulation, in some cases, as much as, e.g., about 90 weight per cent of the formulation. It is preferred that the cellulose derivative in a gel formulation be in the range 1-20% by weight. The important factor is to use at least the minimum amount of cellulose derivative that has a stabilizing effect.

The stabilized compositions of the invention are useful in eye drop formulations, salves for wound healing, gel formulations, foams, and the like. Additional materials such as buffers, preservatives, tonicity adjusting agents, anti-oxidants, other polymers (used, e.g., to adjust viscosity or as extenders), and excipients may be used in the stabilized compositions of the invention. Specific illustrative examples of such other materials include phosphate, citrate, or borate buffers, thimerosal, sorbic acid, methyl or propyl paraben, and chlorobutanol preservatives, sodium chloride and/or sugars to adjust the tonicity, polyvinyl alcohol, poly(acrylic acid) or salts thereof, polyvinyl pyrrolidone, mannitol, lactose, sucrose and ethylene diamine tetra-acetic acid.

The concentration of growth factor in the stabilized composition of the present invention is an effective wound healing amount which is within the range of from about 0.01 to about 1000 micrograms per milliliter of aqueous formulation (that is, either the initial aqueous formulation or a formulation that has been reconsititued after dehydration). Preferably, the growth factor concentration is 1-500 micrograms/ml and more preferably in the range 1-100 micrograms/ml.

Products which may include the cellulose polymer stabilizer of the present invention to stabilize a growth factor preparation include eye drops, eye gels, eye creams, liposome or micell formulations, hydrogels and creams for wound healing such as burn treatment, aqueous vehicles for soaked gauze dressings, burn dressings, artificial skins, suture coatings and products for gastrointestinal indications, such as use in inhibiting gastric acid secretion.

Another product which may include the composition of the present invention is a human mother's breast milk formulation. The breast milk formulation may be used as a substitute for mother's breast milk for nursing infants. Human breast milk has been reported to contain high concentrations of EGF (Read, L.C. et al, Ped. Res. 18: 133-139 (1984)). It has also been reported that recombinant human EGF is indistinguishable from the naturally occurring EGF in mother's milk (Read, L.C. et al., J. Endocrin. 109: 245-250 (1986)). It is believed that the EGF present in mother's milk stimulates infant cell growth and differentiation.

It is contemplated that a dietary milk supplement or breast milk substitute for infants may be prepared by incorporating recombinant derived human EGF into a breast milk formulation. The formulation may be in liquid or dry powder form. If in liquid form, the formulation is stabilized by the stabilizers of the present invention. The concentration of EGF in a liquid formulation may be in the range 0.01-100 micrograms/ml, preferably 0.1 to 10 micrograms/ml. The formulation may also contain nutritional amounts of protein, carbohydrates and fats. The protein should be capable of providing the twenty amino acids essential for human nutrition. The protein may be derived from bovine milk, e.g. casein, or from soybeans.

The aqueous compositions of the present invention may be used in methods of healing wounds. Such methods include incorporating the stabilized aqueous composition into a cream formulation or soaking a gauge dressing with the aqueous solution and then applying the cream or soaked gauze to a wound site, such as a burn, donor site wound, ulcer or any type of cutaneous wound. Additionally, sutures may be coated or soaked with the stabilized aqueous composition and used to close an open wound.

Stable ophthalmic formulations containing EGF may be prepared. The EGF is present in the ophthalmic formulation in a concentration of about 1 to about 1000 micrograms/ml. It is preferred that the EGF concentration be in the range 10-500 micrograms/ml and more preferably that the concentraton be 1-100 micrograms/ml. The formulation additionally contains a water soluble, ophthalmically compatible polymer for maintaining the viscosity of the formulation within the range 1-1000 mPas (cps). The viscosity of the formulation will depend on the polymer, polymer concentration, solvent system and shear rate. Manipulation of these factors may be readily undertaken to achieve the desired viscosity. The viscosity may be measured on a Brookfield viscometer, which measures the force required to "shear" a liquid. The Brookfield method allows for interpolymer interactions (e.g. chain entanglements) and is usually used to measure viscous polymer solutions where the interpolymer interactions play a significant role in the observed viscosity. The results of such measurement are normally reported at a given shear rate (sec$^{-1}$). When the rate is not specified for the viscosity value, it is generally recognized that the rate has been extrapolated to zero. This has been done with the values 1-1000 mPas (cps) given herein. Viscosity values referred to herein are at room temperature, e.g. 22-25°C.

It is important to use high concentrations of EGF in the ophthalmic formulation because simple aqueous solutions containing EGF are quickly drained through the naso-lacrymal duct when placed in a subject's eye. Use of a high concentration of EGF increases the residence time of an effective concentration of the EGF at the wound site in the eye. Also, use of polymers to increase the viscosity of the formulation increases the residence time or contact time of the EGF in the eye by reducing the rate of drainage. The viscosity increasing polymers may be one or more of the stabilizing cellulose polymers of the present invention or may be other water soluble ophthalmically compatible polymers which have viscosity enhancing properties. When a cellulose derivative is used to provide viscosity within the range 1-5000 mPas (cps), the molecular weight should be in the range 80,000 to 240,000.

The polymer may be present in the formulation at a concentration of about 0.05% to about 3.0% (w/v). Any water soluble, ophthalmically compatible polymer which is capable of maintaining the viscosity of the formulation within the range 1-1000 mPas (cps) may be used. Suitable polymers include polysaccharides, such as the cellulose derivatives, vinyl polymers, such as polyvinyl alcohol and polyvinyl pyrrolidone, polyamino acids, such as polylysine, and polyethylene glycol. The polymer is preferred to be one of the stabilizing cellulose derivatives of the present invention and may additionally contain polyvinyl alcohol or polyvinyl pyrrolidone. However, if the formulation is to be stabilized against loss of biological activity, then the polymer should be one of the stabilizing polysaccharides.

The ophthalmic formulation may additionally contain a preservative, at a concentration from about 0.0002% to about 2.5% (w/v). Suitable preservatives are thimerosal (0.0002-0.01%), sorbic acid (0.05-2.5%), chlorobutanol (0.05-0.6%), and EDTA (0.01-0.1%).

The ophthalmic formulation may also contain one or more water soluble extenders for lyophilization, at a concentration of about 0.1-6.0% (w/v). Suitable extenders are mannitol, sucrose, lactose and glycine. Since EGF does not crystallize and does not pack well by itself in lyophilized form, the extenders may be added in order to enhance the packing of the lyophilized EGF into a solid "cake".

The ophthalmic formulation may also contain a buffer for maintaining the pH of the ophthalmic solution between about 5.0 and about 8.0, preferably 7.0-8.0. Suitable buffers are those selected from phosphate buffers, citrate buffers, borate buffers and acetate buffers.

An optional ingredient for the ophthalmic formulation is a tonicity agent for making the aqueous solution isotonic and isosmotic. The tonicity agent may be present in a concentration up to about 1.8% (w/v). Suitable tonicity agents are chloride salts, such as sodium chloride and potassium chloride. The extenders mentioned above, such as mannitol, can also act as a tonicity agent as well as an extender and therefore the tonicity agent may be omitted from the formulation and more of the extender added to achieve a desired tonicity effect.

A method for increasing the rate of healing of a wound in a subject's eye comprises contacting the wound with an effective wound healing amount of the above described ophthalmic formulation. Suitable subjects are primates, such as humans. The ophthalmic formulation may be used in the form of eye drops to increase the rate of healing and maturation of corneal epithelial cells. The formulation may also be used to stimulate growth of mesothelial cells in the eye. Topical application of the ophthalmic formulation in an eye drop may be used to treat epikeratophakia, corneal ulcers, radial keratotomy, corneal transplants and other surgically induced wounds in the eye.

In Example 1 reported below, the biological activity of stabilized and unstabilized aqueous epidermal growth factor formulations was assayed by the receptor binding assay method of Savage et al., Analytical Biochem., 111, pages 195 et seq. (1981). Alternatively, stability may also be measured by any suitable HPLC method. Briefly, the receptor binding assay procedure is the following:

The receptor binding assay is based on the ability of human EGF to compete with $^{125}$I-labeled mouse epidermal growth factor for binding sites on human cells. The binding was performed on confluent monolayers of formalin fixed human epidermal carcinoma A431 cells [Ref. - Fabricant et al., Proc. Natl. Acad. Sci. USA, Vol. 74, p. 565 (1977), Haigler et al., Proc. Natl. Acad. Sci. USA, Vol. 75, p. 3317 (1978), and Ullrich et al., Nature, Vol. 309, p. 418 (1984)] which possess 10-50 times more EGF receptors on their surface than most other cell types. Labelled mouse epidermal growth factor ($^{125}$I-EGF) obtained from Amersham was used.

Multidishes containing a plurality of wells were used. Each well had a confluent monolayer of the formalin fixed human epidermal carcinoma A431 cells affixed to the bottom. First, into each well was added 80 microliters of PBS diluent (phosphate buffered saline containing 0.1 weight per cent bovine serum albumin and 0.2M glycine) containing either known standard epidermal growth factor, the sample to be assayed, or a control containing no epidermal growth factor. Serial dilutions of the standard and sample reagents were usually employed. Next, 20 microliters of the $^{125}$I-EGF of a known activity and concentration in PBS was added to each well. (It is important that the reagent additions be made in the sequence given.) The wells were covered and incubated at 37° C. for about 1-1/4 to 2-1/2 hours.

The reaction mixture in each well was aspirated to discard the liquid, each well was washed twice with PBS, and the wash liquid was discarded. One hundred microliters of 0.1N NaOH, 1 weight per cent sodium dodecyl sulfate, was added to each well. The wells were incubated at 37° C. for 10 minutes, and then the samples were transferred individually to gamma-ray counter vials. The vial with the sample was placed in the gamma ray counter and the number of gamma rays was counted for one minute. Alternatively, multidishes containing removable wells may be employed, in which case, after the washing step, the entire well was removed and placed in the gamma ray counter for counting.

The counts from a serial dilution of a freshly prepared known EGF standard were plotted as a function of EGF concentration on a log-log graph, with the counts per minute being the Y-axis and the concentration being the X-axis. The counts per minute are inversely proportional to the concentration of the known epidermal growth factor. The curves found for the unknown samples at known dilutions were compared against the curve for a freshly prepared known standard to determine the concentration of active EGF (in micrograms per milliliter) in each unknown sample. Values obtained from duplicates and serial dilutions were averaged to improve the accuracy.

Example 1

Stability Studies

Pharmaceutical grade polymers were obtained from the following sources:
Poly(vinyl alcohol) - Gelvatol 40/20 - Monsanto ("PVA")
Methylcellulose - Methocel A4M - Dow ("MC")
Hydroxypropyl methylcellulose-Methocel E4M-Dow ("HPMC")
Poly(vinyl pyrrolidone) - Plasdone C15 - GAF ("PVP")

Four polymer solutions were prepared to give viscosities similar to those currently used in ophthalmic formulations. The solutions also contained 0.01 weight per cent thimerosal to inhibit bacterial growth and 0.9 weight per cent NaCl to make them isotonic. The solutions were filtered through a 0.2 micron polysulfone filter to sterilize them. The filtered solutions were stored in sterile glass vials. Epidermal growth factor produced as described in Bell, WO 85/00369, was added to each vial to provide a solution of 12 micrograms per milliliter. Two control solutions were also used; one contained epidermal growth factor in pure distilled water, and the other contained epidermal growth factor in distilled water containing only the thimerosal plus NaCl. Table 1 displays the concentrations and intrinsic viscosities at 25° C of the four polymer solutions. Intrinsic viscosity is used to define the viscosity of a dilute solution and is usually measured in an Ubbelohde viscometer. Intrinsic viscosity depends on polymer molecular weight and the solvent.

### Table 1

| Polymer | Concentration, wt. % | Intrinsic Viscosity, dl/gm |
|---|---|---|
| PVA | 1.4 | 0.24 |
| MC | 0.25 | 6.86 |
| HPMC | 0.25 | 6.20 |
| PVP | 1.4 | 0.16 |

The epidermal growth factor activities of the six solutions were determined by the receptor binding assay procedure described above for the freshly made solutions, and at 6, 21, and 48 days after preparation. The solutions were stored during the test in the vials at 37° C.

Table 2, below, displays the activities (expressed as micrograms of active epidermal growth factor per milliliter) for the six solutions, both as made and at the 6, 21, and 48 day intervals, and the per cent of the original activity remaining after 48 days.

## Table 2
### Receptor Binding Activity

| Solution | Activities | | | | % of original activity after 48 days |
|---|---|---|---|---|---|
| | Day 0 | Day 6 | Day 21 | Day 48 | |
| Water | 10.6±1.0 | 10.5±1.5 | 10.6±1.2 | 6.6±0.5 | 62 |
| Thimerosal/ NaCl | 12.3±0.7 | 15.3±4.0 | 12.8±1.2 | 6.8±2.1 | 55 |
| PVA | 16.1±3.5 | 14.3±1.1 | 4.3±2.4 | 7.6±0.4 | 47 |
| MC | 18.6±5.0 | 12.3±2.3 | - | 16.8±0.6 | 90 |
| HPMC | 17.4±6.0 | - | - | 21.4±4.0 | 100 |
| PVP | 12.3±4.0 | 10.9±0.1 | 6.1±0.02 | 5.0±1.4 | 41 |

Certain polymers caused the EGF concentration values to be measured artifically high. Therefore, concentration values should not be compared between different polymers. The concentration values must be compared between Day 0 and Day 48 for each individual polymer. As can be seen by the results displayed above, the aqueous epidermal growth factor solutions containing the two cellulose derivatives lost none of their biological activity (within experimental error) after storage for 48 days, whereas the others lost approximately one-half of their biological activity after the same period of time.

Example 2

Evaluation of Ophthalmic EGF Formulations in Primates

This example demonstrates the effect of human EGF on accelerating epithelial regeneration of primate corneas. The test formulation utilized contained human EGF produced by recombinant methods (Chiron Corp., Emeryville, CA) at a concentration of 100 micrograms/ml. The formulation used was prepared at pH 5.5 with chlorobutanol (Chlorobutanol 8A) as a preservative and the following formulation:

| Ingredients | % (w/v) |
|---|---|
| NaCl | 0.46 |
| Chlorobutanol | 0.50 |
| Citric Acid | 0.26 |
| Na Citrate.H20 | 0.57 |
| Mannitol | 1.0 |
| HPMC | 0.25 |
| Water | 100 |

Three adult female Macaca fascicularis primates weighing five to seven kilograms were used. The anatomical structure of primate and human corneas are nearly identical, and primates are a well accepted model for epithelial regeneration studies.

Each animal was anesthetized with Ketamine (5 mg/lb = 11 mg/kg) and Rompum (1 mg/lb = 2.2 mg/kg). Atropine sulphate (0.5 mg/lb = 1.1 mg/kg) was also given to reduce salivation. A lid speculum was

7

inserted in the eye and a stainless steel vacuum trefine with an 8 mm diameter was attached to the cornea. The center of the trefine was filled with n-heptanol for 45 seconds to remove the epithelium. Then, the n-heptanol was removed by aspiration and the cornea was flooded with 30 milliliters of phosphate buffered saline. The epithelium was removed to the limbus by gently wiping the surface with a cotton-tipped applicator. Epithelial removal was confirmed by fluorescein staining. The animals were treated four times each day at 09:00, 12:00, 15:00, and 18:00 hours with 2 drops of solution. The left eye served as a control and received vehicle only (i.e. all ingredients except EGF) while the right eye received vehicle containing EGF. The primates were anesthetized daily, as described above, their corneas stained with fluorescein and then photographed with color slide film.

The extent of epithelial regeneration was measured by computer-assisted planimetry of enlarged projections of the color slides using the Sigma-Scan program and was expressed as the percent of each cornea which had re-epithelized. The results were analyzed for statistical significance by a paired T-test. The primates were humanely euthanized at the end of the experiment and their corneas processed for routine histology.

The results of the computer-aided planimetry of the enlarged color slides is shown in Table 3. As can be seen, the formulation provided increased area of epithelial regeneration 24 hours after injury. Statistical analysis of the results at 24 hours using one-tailed paired-T test indicated a small p-value of $0.10 > p > 0.05$. Primate No. 11316 showed a slight reversal in epithelialized area from 48 to 72 hours (92.5% to 88.2%) which recovered to a punctuate defect during the next 24 hours.

Histologic evaluation of the corneas from primates Nos. 11314 and 10668 treated with the EGF containing formulation revealed a thick, continuous epithelium of approximately 5-7 cell layers thick. The cells containing the basal layer were cuboidal and compact. Cells overlying the basal layer showed progressive stratification to squamous epithelium. In comparison, control corneas from these primates had very thin epithelium, approximately 2-3 cells thick with widely spaced and somewhat flattened basal and poor stratification of overlying cells. The appearance of both corneas from primate No. 11316 was similar to control corneas of primates Nos. 11314 and 10668.

Thus, it is readily apparent that treatment of corneal epithelial defects with the EGF containing formulation accelerated the early rate of epithelial regeneration compared to vehicle. Also, the histological appearance of the regenerated epithelium was superior on corneas treated with EGF. Thus, the EGF formulation improved both the rate and the quality of regenerated epithelium in this primate model.

## Table 3
### Computer Planimetry of Re-Epithelialized Areas from Photographs

| Monkey# | Eye | 0 Hr. | 24 Hr. | 48 Hr. | 72 Hr. | 96 Hr. |
|---------|-----|-------|--------|--------|--------|--------|
| 11314 | L | 0 | 49.5 | 90.4 | 100 | 100 |
|  | R | 0 | 59.3 | 94.4 | 100 | 100 |
| 11316 | L | 0 | 31.4 | 75.6 | 96.2 | 100 |
|  | R | 0 | 50.5 | 92.5 | 88.2 | 97.1 |
| 10668 | L | 0 | 48.6 | 88.1 | 95.2 | 100 |
|  | R | 0 | 50.1 | 83.1 | 96.3 | 100 |

Example 3

In addition to the chlorobutanol 8A formulation referred to in Example 2, two other formulatins were also prepared.

Formulation 6 was prepared at pH = 7.0 and contains thimerosal as a preservative.

| Ingredients | Thimerosal 6A % (w/v) |
|---|---|
| NaCl | 0.51% |
| Thimerosal | 0.004 |
| EDTA | 0.1 |
| NaH2PO4.H20 | 0.18 |

| Ingredients | Thimerosal 6A % (w/v) |
|---|---|
| Na2HPO4.7H2O | 0.54 |
| Mannitol | 1.0 |
| Hydroxy propyl Methylcellulose E4M | 0.25 |
| Water | 100 |

Formulation 7 was prepared at pH = 6.65 and contains sorbic acid as a preservative.

| | Sorbic 7A % (w/v) |
|---|---|
| NaCl | 0.50 |
| Sorbic acid | 0.20 |
| EDTA | 0.1 |
| Citric acid | 0.26 |
| Na citrate.2H20 | 0.57 |
| Mannitol | 1.0 |
| HPMC | 0.25 |
| water | 100 |

The stability of the three different formulations was determined over a 292 day period and at four different temperatures. The results are listed in Table 4.

## Table 4

### STABILITY OF OPHTHALMIC FORMULATIONS

The EGF concentration of these samples was prepared as 10 ug/ml.

| Formulation | 0 | 15 | 30 | 93 | 124 | 292 |
|---|---|---|---|---|---|---|
| | | | Time (Days) | | | |
| Thimerosal-6A | | | | | | |
| 4C | 7.68 | 8.54 | 10.1 | 8.5 | 10.2 | 9.4 |
| 22C | " | 8.76 | 10.1 | 7.0 | 8.0 | -- |
| 37C | " | 8.82 | 8.8 | 3.8 | -- | -- |
| 45C | " | 6.88 | 6.3 | ND | -- | -- |
| Sorbic-7A | | | | | | |
| 4C | 7.53 | 10.0 | 8.6 | 7.1 | 6.9 | 3.2 |
| 22C | " | 6.72 | 7.4 | 4.1 | -- | -- |
| 37C | " | 4.94 | 5.2 | ND | -- | -- |
| 45C | " | 3.12 | 2.7 | ND | -- | -- |
| Chlorobutanol-8A | | | | | | |
| 4C | 7.49 | 10.24 | 11.3 | 7.7 | 9.9 | 9.3 |
| 22C | " | 8.14 | 9.8 | 2.6 | -- | -- |
| 37C | " | 6.28 | 7.0 | 2.3 | -- | -- |
| 45C | " | 4.45 | 3.6 | 2.4 | -- | -- |

ND= not detectable

Example 4

It is contemplated that a human mother's breast milk formulation may be prepared which incorporates recombinantly produced human EGF. Such a formulation may be prepared as a liquid or in a dry powder form. If in liquid form, it is desirable to incorporate the water soluble polysaccharide stabilizers of the present invention to stabilize the EGF against loss of biological activity. The stabilizers will increase the shelf life of the breast milk formulation.

The formulation contains any of the nutritional ingredients commonly incorporated into milk or breast milk substitutes. Such ingredients include nutritional amounts of protein, carbohydrates and fats, to provide essential amino acids and calories for the infant. The protein should be capable of providing all 20 amino acids which are essential for human nutrition. The protein may be derived from bovine milk, e.g. casein, or from soybeans if an animal protein-free formulation is desired. The carbohydrates may include lactose, or if a lactose-free product is desired, any other suitable carbohydrate may be used, such as sucrose or fructose.

The formulation may also include nutritional amounts of compounds for providing the following vitamins: A, D, K, E, C, $B_1$, $B_2$, $B_6$, $B_{12}$, Niacin, Folic Acid, Pantothenic Acid, Biotin, Choline, and Inositol, either singly or in any combination.

Additionally, the formulation may include nutritional amounts of compounds providing minerals such as: calcium, phosphorus, magnesium, iron, iodine, zinc, copper, manganese, sodium, potassium, or chloride, either singly or in any combination.

The invention has been described herein with reference to certain preferred embodiments and examples. However, since obvious variations will appear to those skilled in the art, the invention is not to be considered limited thereto but only by the claims which follow.

## Claims

1. An aqueous medicinal composition containing a growth factor selected from epidermal growth factor, transforming growth factor-alpha, transforming growth factor-beta, basic fibroblast growth factor, acidic fibroblast growth factor, angiogenin, nerve growth factor, insulin-like growth factor, platelet derived growth factor or a mixture thereof and an amount of a water-soluble cellulose polymer sufficient to stabilise said growth factor against loss of biological activity.

2. The composition of claim 1, wherein the growth factor is epidermal growth factor.

3. The composition of claim 2, wherein the epidermal growth factor is human epidermal growth factor.

4. The composition of any one of claims 1 to 3, wherein the cellulose derivative is hydroxypropyl cellulose, methylcellulose, hydroxyethyl cellulose, hydroxypropyl methylcellulose or a mixture thereof.

5. A method for stabilising an aqueous medicinal composition containing a growth factor selected from epidermal growth factor, transforming growth factor-alpha, transforming growth factor-beta, basic fibroblast growth factor, acidic fibroblast growth factor, angiogenin, nerve growth factor, insulin-like growth factor, platelet derived growth factor or a mixture thereof, which method comprises incorporating in said composition an amount of a water-soluble cellulose polymer sufficient to stabilise said growth factor against loss of biological activity in the presence of water.

6. A pharmaceutically compatible cream or gel comprising the composition of any one of claims 1 to 4.

7. A suture or an absorbent gauze dressing coated or soaked with the composition of any one of claims 1 to 4.

8. The composition of any one of claims 1 to 4, the cream or gel of claim 6, or the suture or gauze dressing of claim 7 for use in increasing the rate of healing of a wound.

9. A mother's breast milk substitute comprising the composition of any one of claims 1 to 4.

10. A mother's breast milk substitute according to claim 9 comprising recombinant human EGF and a nutritional amount of protein capable of providing the amino acids essential for human nutrition.

## Patentansprüche

1. Wäßrige medizinische Zusammensetzung, enthaltend einen Wachstumsfaktor, ausgewählt unter Epidermis-Wachstumsfaktor, Umwandlungs-Wachstumsfaktor-alpha, Umwandlungs-Wachstumsfaktor-beta, basischem Fibroblasten-Wachstumsfaktor, saurem Fibroblasten-Wachstumsfaktor, Angiogenin, Nerven-Wachstumsfaktor, Insulin-ähnlichem Wachstumsfaktor, von Blutplättchen abgeleitetem Wachstumsfaktor oder einem Gemisch davon, und eine Menge an wasserlöslichem Cellulose-Polymer, ausreichend, um den genannten Wachstumsfaktor gegen den Verlust der biologischen Aktivität zu

EP 0 267 015 B1

stabilisieren.

2. Zusammensetzung nach Anspruch 1, in der der Wachstumsfaktor ein Epidermis-Wachstumsfaktor ist.

3. Zusammensetzung nach Anspruch 2, in der der Epidermis-Wachstumsfaktor ein Human-Epidermis-Wachstumsfaktor ist.

4. Zusammensetzung nach einem der Ansprüche 1 bis 3, in der das Cellulose-Derivat Hydroxypropylcellulose, Methylcellulose, Hydroxypropylmethylcellulose oder ein Gemisch davon ist.

5. Verfahren zum Stabilisieren einer wäßrigen medizinischen Zusammensetzung, enthaltend einen Wachstumsfaktor, ausgewählt unter Epidermis-Wachstumsfaktor, Umwandlungs-Wachstumsfaktor-alpha, Umwandlungs-Wachstumsfaktor-beta, basischem Fibroblasten-Wachstumsfaktor, saurem Fibroblasten-Wachstumsfaktor, Angiogenin, Nerven-Wachstumsfaktor, Insulin-ähnlichem Wachstumsfaktor, von Blutplättchen abgeleitetem Wachstumsfaktor oder einem Gemisch davon, wobei das genannte Verfahren umfaßt das Einbringen einer Menge an wasserlöslichem Cellulose-Polymer in die genannte Zusammensetzung, ausreichend, um den genannten Wachstumsfaktor gegen den Verlust der biologischen Aktivität in Gegenwart von Wasser zu stabilisieren.

6. Pharmazeutisch verträgliche(s) Creme oder Gel, die Zusammensetzung nach einem der Ansprüche 1 bis 4 umfassend.

7. Nahtmaterial oder absorbierender Mullverband, beschichtet oder getränkt mit der Zusammensetzung nach einem der Ansprüche 1 bis 4.

8. Zusammensetzung nach einem der Ansprüche 1 bis 4, Creme oder Gel nach Anspruch 6, oder Nahtmaterial oder Mullverband nach Anspruch 7 zur Verwendung bei der Bechleunigung der Heilung einer Wunde.

9. Muttermilch-Ersatz, die Zusammensetzung nach einem der Ansprüche 1 bis 4 umfassend.

10. Muttermilch-Ersatz nach Anspruch 9, umfassend rekombinanten Human-EGF und eine Nahrungsmenge an Protein, welche die für die menschliche Ernährung essentiellen Aminosäuren zur Verfügung stellen kann.

**Revendications**

1. Composition médicinale aqueuse contenant un facteur de croissance choisi parmi le facteur de croissance épidermique, le facteur alpha de croissance des transformations, le facteur bêta de croissance des transformations, le facteur de croissance des fibroblastes basique, le facteur de croissance des fibroblastes acide, l'angiogénine, le facteur de croissance des cellules nerveuses, le facteur de croissance analogue à l'insuline, le facteur de croissance dérivé des plaquettes sanguines, ou un mélange de ceux-ci, et une quantité d'un polymère de cellulose hydrosoluble suffisante pour stabiliser ledit facteur de croissance contre une perte d'activité biologique.

2. Composition selon la revendication 1, dans laquelle le facteur de croissance est du facteur de croissance épidermique.

3. Composition selon la revendication 2, dans laquelle le facteur de croissance épidermique est du facteur de croissance épidermique humain.

4. Composition selon l'une quelconque des revendications 1 à 3, dans laquelle le dérivé de cellulose est de l'hydroxypropyl cellulose, de la méthylcellulose, de l'hydroxyéthyl cellulose, de l'hydroxypropyl méthylcellulose, ou un mélange de celles-ci.

5. Procédé pour la stabilisation d'une composition médicinale aqueuse contenant un facteur de croissance choisi parmi le facteur de croissance épidermique, le facteur alpha de croissance des transformations, le facteur bêta de croissance des transformations, le facteur de croissance des fibroblastes basique, le

12

facteur de croissance des fibroblastes acide, l'angiogénine, le facteur de croissance des cellules nerveuses, le facteur de croissance analogue à l'insuline, le facteur de croissance dérivé des plaquettes sanguines, ou un mélange de ceux-ci, lequel procédé comprend l'incorporation dans ladite composition d'une quantité d'un polymère de cellulose hydrosoluble suffisante pour stabiliser ledit facteur de croissance contre une perte d'activité biologique en présence d'eau.

6. Crème ou gel pharmaceutiquement compatible comprenant la composition selon l'une quelconque des revendications 1 à 4.

7. Suture ou pansement de gaze absorbante revêtu ou imprégné avec la composition selon l'une quelconque des revendications 1 à 4.

8. Composition selon l'une quelconque des revendications 1 à 4, crème ou gel selon la revendication 6, ou suture ou pansement de gaze selon la revendication 7 pour une utilisation pour l'accroissement de la vitesse de cicatrisation d'une blessure.

9. Substitut du lait du sein maternel comprenant la composition selon l'une quelconque des revendications 1 à 4.

10. Substitut du lait du sein maternel selon la revendication 9, comprenant de l'EGF humain recombinant et une quantité nutritionnelle de protéine apte à fournir les acides aminés essentiels pour la nutrition humaine.